Europäisches Patentamt

(19) European Patent Office

Office européen des brevets

(11) EP 0 781 558 A2

(12) EUROPEAN PATENT APPLICATION

(43) Date of publication:
02.07.1997 Bulletin 1997/27

(51) Int. Cl.$^6$: A61K 38/57

(21) Application number: 96120816.2

(22) Date of filing: 23.12.1996

(84) Designated Contracting States:
BE CH DE DK ES FR GB IT LI NL SE

(30) Priority: 27.12.1995 JP 341789/95

(71) Applicants:
• Tsukada, Minoru
  Hirakata, Osaka 573 (JP)
• Matsui, Tomohiko
  Osaka 541 (JP)
• Shintome, Masakazu
  Hirakata, Osaka 573 (JP)
• Tsuchiyama, Hiromi
  Hirakata, Osaka 573 (JP)
• Maruyama, Tomoyuki
  Hirakata, Osaka 573 (JP)

(72) Inventors:
• Tsukada, Minoru
  Hirakata, Osaka 573 (JP)
• Matsui, Tomohiko
  Osaka 541 (JP)
• Shintome, Masakazu
  Hirakata, Osaka 573 (JP)
• Tsuchiyama, Hiromi
  Hirakata, Osaka 573 (JP)
• Maruyama, Tomoyuki
  Hirakata, Osaka 573 (JP)

(74) Representative: VOSSIUS & PARTNER
Siebertstrasse 4
81675 München (DE)

(54) **Pharmaceutical use of heparin cofactor II**

(57) Described is a pharmaceutical composition comprising heparin cofactor II (HCII) as active ingredient. Described is also the use of this pharmaceutical composition as a preventative and therapeutic agent for sepsis, thrombosis, incomplete microcirculation, ischemic disease, organ derangement in liver, lungs, kidney, brain etc., and various inflammations. Also described is a pharmaceutical composition as mentioned above which further comprises AT III. The pharmaceutical composition is effective for prevention and therapy of sepsis, a disease in which an abnormal vascular flow arises, organ derangement in liver, lungs, kidney, brain, etc. and various inflammations.

EP 0 781 558 A2

## Description

The present invention relates to a pharmaceutical composition having heparin cofactor II as active ingredient. More particularly, the invention relates to the pharmaceutical composition above which is a preventive and therapeutic agent of sepsis, a disease in which an abnormal vascular flow arises, organ derangement in liver, lungs, kidney, brain, etc. and various inflammations. Still more particularly, the invention relates to the pharmaceutical composition above prepared by further compounding antithrombin III.

There are substances having a coagulation inhibitor activity in blood. Heparin cofactor II (hereinafter abbreviated as HCII) is a plasma glycoprotein purified by D. M. Tollefsen et al. (J. Biol. Chem., Vol. 257, 2162-2169, 1982) which is known to mainly inhibit thrombin in the presence of dermatan sulfate and/or a large quantity of heparin (D. M. Tollefsen et al., J. Biol. Chem., Vol. 258, 6713-6716, 1983). Other proteins which have an antithrombin action include antithrombin III (hereafter abbreviated as ATIII). It has been known that ATIII plays an important role in the antithrombin action in vitro from the high onset frequency of thrombosis in ATIII defective patients, and ATIII has already been applied to clinical use. However, there is no case where the effect of in vivo administration of HCII is investigated, and the action of HCII in vivo is still unknown.

Thus, the technical problem underlying the present invention is to reveal the biological action of HCII in vivo as well as to provide a pharmaceutical composition having this substance as an active ingredient, particularly the pharmaceutical composition above which is a preventive and therapeutic agent of sepsis, a disease in which an abnormal vascular flow arises, organ derangement in liver, lungs, kidney, brain, etc. and various inflammations.

The technical problem has been solved by providing the embodiments characterized in the claims.

As a result of experiments in which HCII was administered to various experimental disease animal models, it was found that HCII alone indicates remarkable preventative and therapeutic activity against sepsis, various thromboses, incomplete microcirculation, ischemic disease, liver function failure, incomplete respiration, kidney function failure, cerebral disorder and various inflammations without simultaneous administration of dermatan sulfate and/or heparin. Moreover, the success in further increasing the preventive and therapeutic effects by the combined use of HCII and ATIII completed the present invention.

Thus, the present invention relates to a pharmaceutical composition comprising HCII as an active ingredient. Preferably, this pharmaceutical composition is a preventative and therapeutic agent of the following diseases in 1)-6):

1) infectious disease, particularly sepsis,
2) thrombosis, e.g. disseminated intravascular coagulation syndrome (hereafter abbreviated as DIC), intraarterial and intravascular thrombus, thrombus in an organ, postoperative thrombosis, thrombosis associated with angiopachynsis, acute stricture or restenosis, etc. after PTCA or PTCR,
3) incomplete microcirculation,
4) ischemic disease, e.g. ischemic brain syndrome, ischemia due to operation, etc.
5) liver function failure, pulmonary function failure, e.g. acute respiratory distress syndrome (ARDS) and idiopathic respiratory distress syndrome (IRDS), etc., kidney function failure, e.g. nephrotic syndrome, etc., cerebral function failure, etc., and
6) inflammation, e.g. glomerular nephritis, pancreatitis, inflammation associated with edemaplasia, etc.

In a preferred embodiment, the present invention relates to a pharmaceutical composition as described above for administering 1-10000 unit/kg-body-weight/day in a form for intravenous administration, intraarterial administration or intratracheal administration. In a further preferred embodiment, the pharmaceutical composition according to the invention furthermore comprises ATIII.

FIG. 1 is a graph showing the influence of dose on the intrahepatic thrombus formation inhibition effect of HCII and ATIII;
FIG. 2 is a graph showing the fluctuation of blood HCII in a peripheral circulation failure model to which lauric acid is administered; and
FIG. 3 is a graph showing the fluctuation of blood HCII in a renal blood vessel delegation model.

The HCII used in the present invention is not particularly limited, so far as derived from a human and purified to a degree available for a drug, and both natural and artificial ones will do.

Natural HCII is purified, for example, from human whole blood, plasma, serum or the serum expressed from clotted blood, and so on. For the method for purification of HCII, the method of Blinder et al. (J. Biol. Chem., Vol. 264, 5128-5133, 1989) is exampled.

As for artificial HCII (e.g. Blinder et al., Biochemistry, 27: 752-759, 1988), the one produced by culturing the host cells transformed by the recombination vector containing the DNA coding the variant HCII whose antithrombin activity is retained or improved (e.g. refer to Japanese Patent Application Laid-Open No. 3-139280), the one produced by chemical synthesis, etc.

The pharmaceutical composition of the present invention can be properly mixed with the components necessary for pharmaceutical preparation such as pharmaceutically acceptable additives (e.g. support, excipient and diluting agent), etc. and administered orally or non-orally in an embodiment such as a liquid preparation, powder, granule, tablets, capsule, syrup and injection. It is particularly preferable to prepare HCII together with pharmaceutically acceptable additives as a lyophilized product in order to provide the preparation which may be dissolved with a suitable vehicle at use or a liquid preparation. A lyophilized preparation is diluted, for example, with distilled water for injection to prepare a ca. 1-1000 unit/m$\ell$ solution of HCII, and more preferably, its salt concentration is adjusted to a physiologically isotonic concentration before administration.

The pharmaceutical composition of the present invention can be administered in an administration form such as oral, intravenous, intraarterial administration and administration in airway. Preferably, intravenous, intraarterial administration and administration in airway are exampled.

The dose of the pharmaceutical composition of the present invention, which varies in accordance with the degree of the progress of the disease, how serious the patient (patient animal) is affected by a specific disease, tolerance to the drug, weight, species of the animal, is generally 1-10000 unit/kg-body-weight/day, preferably 10-2000 unit/kg-body-weight/day for an adult, and the dose can be administered all at once or over several occasions. (The titration of HCII was performed by measuring the antithrombin activity of HCII in the presence of heparin with the synthetic substrate method, preparing a calibration curve using the ATIII whose titer is known, and making one unit of ATIII as one unit of HCII. One unit of ATIII corresponds to the amount of ATIII contained by 1 m$\ell$ of normal human plasma.)

A more preferable embodiment of the pharmaceutical composition of the present invention is one in combination with ATIII. ATIII may be compounded in an HCII preparation, or single preparations of respective agents may be administered sequentially or simultaneously.

In the case of separate preparations, ATIII can be properly mixed with the components necessary for pharmaceutical preparations, such as pharmaceutically acceptable additives (e.g. support, excipient and diluting agent), etc. and administered orally or non-orally in an embodiment such as a liquid preparation, powder, granule, tablets, capsule, syrup and injection. It is particularly preferable to prepare ATIII together with pharmaceutically acceptable additives as lyophilized product in order to provide a preparation which may be dissolved with a suitable vehicle at use or a liquid preparation. A lyophilized preparation is diluted, for example, with distilled water for injection to prepare a ca. 1-1000 unit/m$\ell$ solution of ATIII, and more preferably, its salt concentration is adjusted to a physiologically isotonic concentration before administration.

The dose in case of combined use of HCII and ATIII, which varies in accordance with the degree of the progress of the disease, how serious the patient (patient animal) is affected by a specific disease, tolerance to the drug, weight, species of the animal, is generally 1-10000 unit/kg-body-weight/day, preferably 10-2000 unit/kg-body-weight/day for an adult, and the dose can be administered all at once or over several occasions.

Preferably, the pharmaceutical composition of the present invention is administered just after the onset of a disease or before the onset of a disease when there is a possibility of onset. It is also preferable to administer a suitable amount day after day until cure. In case of a disease with poor prognosis, administration may be further continued for a period after cure. It may also be administered from day to day for 1-180 days from before, during or just after completion of an operation if an ischemia due to the operation occurs or a postoperative restenosis, etc. is predicted.

HCII which is the active ingredient of the pharmaceutical composition of the present invention is effective for prevention and therapy of sepsis, various thromboses, incomplete microcirculation, ischemic disease, organ derangement in liver, lungs, kidney, brain, etc. and various inflammations in mammals such as human, dog, cattle, horse, goat, sheep, rabbit, mouse and rat.

Sepsis, caused by infection of various bacteria, is a disease of poor prognosis which coincides with various thromboses such as DIG, the blood flow failure such as incomplete microcirculation, accentuation of membrane permeability in hepatic cells or a blood vessel and a multi-organ failure such as liver function failure or lung failure.

Thrombosis is a disease in which a blood vessel is obstructed by thrombus i.e. blood clot, and it is exampled by DIG, intraarterial and intravenous thrombi, thrombus in an organ, postoperative thrombosis, thrombosis associated with angiopachynsis and restenosis after PTCA or PTCR.

A disseminated intravascular coagulation (DIC) is a syndrome which has an extreme blood coagulation accentuation and systemic vascular multiple thrombi. A postoperative thrombosis is a deep vein thrombosis or a pulmonary vascular occlusion which is frequently formed by operation. As for a thrombosis associated with angiopachynsis, an intracoronary thrombosis is named. As for the therapy for the disease, PTCA (a method for recanalization by direct vasodilation using a balloon Dyler catheter against coronary arteriostenosis) or PTCR (a method for thrombolysis by direct administration of urokinase or streptokinase to the inside of coronary artery) is effective, but it has a poor diagnosis and a high probability of restenosis.

An incomplete microcirculation is an aggravation of blood circulation ranging from arteriola through capillary blood vessel to venula exampled by angiitis such as Buerger's disease and blood stasis concerned by thrombus or vasoconstriction.

EP 0 781 558 A2

Formation of thrombus in an artery or vein can be evaluated, for example, by measuring clotting factor parameters such as blood fibrinogen concentration, clotting time, TAT and D-dimer value. Thrombosis or incomplete microcirculation in an organ (aggravation of organ vascular flow) can be evaluated, for example, by injecting the substances labeled by a radioactive isotope such as $^{125}$I or $^{51}$Cr to a blood vessel and measuring the radioactivity per unit weight of respective organs against the radioactivity per unit volume of blood.

An ischemia is a severe local anemic state, often occurs in a heart, kidney, brain and extremities. An ischemic disease shows cell damage or incomplete cellular function caused by ischemia. As a cause of ischemia, vascular flow cessation by an operation as well as stenosis or occlusion of arterial lumen is named.

A liver is an organ which covers most of the metabolism and its control and further performs detoxification by oxidation, reduction and conjugation, etc. of endogenous and exogenous substances, synthesis of urea, and so on. A liver function failure can be evaluated, for example, by measuring organ failure parameters such as blood GOT, blood urea nitrogen (BUN), blood hyaluronic acid and arterial blood ketone body ratio.

An ARDS is the pulmonary failure disease in which a respiratory failure is brought about by a hemorrhagic or bacterial shock, severe external injuries and drug poisoning. An IRDS is the disease in which the absence of a pulmonary surfactant in air vesicles due to immaturity of the lung leads the ventilation failure of the air vesicles. These alveolar diseases can be evaluated, for example, by arterial blood gas analysis, etc. as well as a histological investigation of air vesicles.

A nephrotic syndrome is a kidney disease in which profuse serum protein components are lost into urine, and the tendency of thrombus formation increases at the same time. A kidney function failure can be evaluated, for example, by measuring the urinary protein concentration or the volume of abdominal dropsy.

An ischemic cerebral disorder has symptoms such as cerebral edema, nervous disorder and incomplete cerebral vascular flow. The degree of cerebral disorder is evaluate, for example, by measuring water content of brain, neurologic defect and cerebral vascular flow.

A glomerular nephritis is the inflammatory disease of glomerulus that generally shows a cell proliferative change, and the intervention of immune response to hemolytic streptococcal infection is suggested as one of the causes. An acute pancreatitis is a disease in which the pancreatic tissue is digested by autolysis with chymotrypsin which is a digestive enzyme in the pancreas. It is reported that HCII inhibits not only thrombin but also chymotrypsin (F.C. Church et al., Proc. Natl. Acad. Sci. USA, 82: 6431-6434, 1985). In addition, the inflammation associated with edemaplasia is caused by the accumulation and pooling of extracellular fluid due to inflammatory stimulus.

A glomerular nephritis can be evaluated, for example, by measuring the increase of extracellular matrix, urinary protein concentration and BUN. A pancreatitis can be evaluated by measuring abdominal dropsy volume as well as the histological investigation of pancreatic tissue lesions. In addition, the inflammation associated with edemaplasia can be evaluated using the enlargement of edema as an index.

The present invention will be further illustrated by the following experimental and reference examples.

The HCII and ATIII used in the following examples were prepared as lyophilized preparation respectively with the method described in Blinder et al., J. Biol. Chem., 264: 5128-5133, (1989) and the method described in Japanese Patent Application Laid-Open No. 6-256213, and both were diluted to a desired concentration with a physiologic saline at use before administration.

Reference example 1. Anticoagulant activity of HCII in vitro

HCII (final concentration 10 unit/m$\ell$) was added to SD rat pooled plasma, then actin and sodium calcium were added to it and the clotting time (aPTT) was measured using a clotting time measuring device (available from Amelung). Adding a physiologic saline instead, the control group was prepared.

As a result, no prolongation of clotting time by the addition of HCII was observed (Table I). It was indicated from the fact that HCII does not show anticoagulative activity (antithrombin activity) in the absence of dermatan sulfate or heparin.

Table 1

| Anticoagulant activity of HCII in vitro in the absence of dermatan sulfate and heparin | |
| --- | --- |
| Administration group | Clotting time (second) |
| Control (saline) group | 17.55±0.25[1] |
| HCII administration group | 18.05±0.65 |

[1] Average of two experiments ± deviation

4

Next, HCII was administered to experimental animal models of sepsis, diseases in which abnormal vascular flow arises, organ derangement in an organ such as liver, lung, kidney or brain, and various inflammations to investigate the effects of HCII in vivo.

Experimental example 1. Effect of HCII in sepsis model

Experiment 1. Survival rate improving effect

SD rats (male, 7-8 week of age) were separated into three groups (Group H, A and H/A) and HCII (500 unit/kg-body-weight), ATIII (500 unit/kg-body-weight) and mixed solution of HCII and ATIII (250 unit/kg-body-weight each) were intravenously administered respectively in Group H, A and H/A, then E. coli-derived endotoxin (10 mg/kg-body-weight, hereafter abbreviated as LPS) was administered intravenously. Then the rats were fed under usual conditions and whether alive or dead was observed after one week. A physiological saline was administered instead of HCII and ATIII in a control group (similar in descending experimental examples, that is called as Saline Group hereafter).

As a result, 6 out of 16 rats survived in Group H whereas only 1 out of 16 rats survived in Saline Group, showing improvement of survival rate. Almost the same effects were observed in the group of only HCII administration and the group of only ATIII administration (7 out of 16 rats). In Group H/A, 10 out of 16 rats survived, indicating that the combined use of HCII and ATIII is more effective.

Experiment 2. Effect of inhibiting thrombus in organ and intravenous and intraarterial thrombi and effect of inhibiting liver function failure

Sodium iodide then $^{125}$I-labeled fibrinogen was administered intravenously to SD rats (male, 7-8 week of age). They were separated into three groups (Group H, A and H/A) and HCII (500 unit/kg-body-weight), ATIII (500 unit/kg-body-weight) and mixed solution of HCII and ATIII (250 unit/kg-body-weight each) were intravenously administered respectively in Group H, A and H/A, then LPS (5 mg/kg-body-weight) was administered intravenously. After 3 hours, pentobarbital was administered intraperitoneal for anesthesia, cardiac blood was collected (citric acid blood collection), then artery was cut to draw all the blood. Lungs, liver, pancreas, spleen, kidney, small intestine and abdominal artery were extirpated and the radioactivity of respective organs and blood were measured by a gamma-counter. The T/B value (Equation 1) was calculated for each rat and the results were compared with each other.

$$T/B = \frac{\text{(radioactivity per unit weight of each organ)}}{\text{(radioactivity per unit volume of blood)}} \qquad \text{[Equation 1]}$$

As a result, the T/B values in Group H were lower than those in Saline Group with all the investigated organs, and it was indicated that HCII has an effect of inhibiting thrombus in organ (Table 2). Similar effect was obtained by administration of ATIII but the degree of inhibition was weaker than that of HCII. In the artery, the degree of T/B values in Group A were similar to those in Saline Group while they showed low values close to normal rats in Group H. This suggests that HCII plays an important role in inhibiting formation of thrombus on the blood vessel wall. Also, the effect of combined use was recognized as the T/B values with lungs, liver, pancreas, spleen, small intestine and kidney in Group H/A were lower than those in the single administration groups. In addition, histological tests were performed with all the organs, and improvement of histological findings were recognized in all of them.

5

Table 2

| Inhibitory effects of HCII and ATIII in formation of thrombus (T/B) in organs of rats to which endotoxin is administered | | | | | | | |
|---|---|---|---|---|---|---|---|
| Drug Group | Lung | Liver | Pancreas | Spleen | Small intestine | Kidney | Artery |
| Normal rat | 0.309± 0.010 | 0.263± 0.005 | 0.160± 0.009 | 0.237± 0.010 | 0.257± 0.015 | 0.316± 0.011 | 0.258± 0.033 |
| Saline | 0.385± 0.017 | 0.742± 0.060 | 0.317± 0.060 | 0.570± 0.032 | 0.375± 0.014 | 0.392± 0.019 | 0.352± 0.042 |
| A | 0.344± 0.011 | 0.420± 0.042 | 0.262± 0.038 | 0.403± 0.037 | 0.358± 0.037 | 0.341± 0.009 | 0.281± 0.032 |
| B | 0.367± 0.019 | 0.494± 0.033 | 0.284± 0.028 | 0.450± 0.062 | 0.339± 0.018 | 0.366± 0.014 | 0.417± 0.038 |
| C | 0.334± 0.008 | 03359± 0.019 | 0.290± 0.048 | 0.314± 0.037 | 0.322± 0.010 | 0.323± 0.012 | 0.320± 0.032 |
| A:HCII 500 unit/kg-body-weight<br>B:ATIII 500 unit/kg-body-weight<br>C:HCII+ATIII 250 unit/kg-body-weight each | | | | | | | |

Some of the collected blood was centrifuged to obtain plasma, and the thus obtained plasma was served for measuring its organ derangement parameters [blood GOT, blood urea nitrogen (BUN), hemoglobin, urinary protein, blood hyaluronic acid and arterial blood ketone body ratio], blood platelet count, white blood cell count, clotting factor parameters (fibrinogen, aPTT, PT, TAT and D-dimer) and cytokine (TNF, IL-1 and IL-8). Table 3 shows a part of the results. In all the investigated items, improving effect was recognized in the three administration groups compared with Saline Group, and the order of the effects was Group H/A > H > A. It was suggested that HCII may be effective for inhibiting liver function failure as the inhibition of the increase of blood GOT level was observed. It was also suggested that HCII is effective not only for inhibition of thrombus formation in an organ but also the thrombus formation in vein and artery from the effect of inhibiting decrease of blood fibrinogen. Moreover, HCII was indicated to be effective for liver function failure and liver deficiency as improvements of blood hyaluronic acid which is an index for the metabolic function of liver endothelial cells present in the liver sinusoid and blood ketone body ratio which is an index for metabolic rotation of TCA circuit in hepatocyte mitochondria were recognized.

Table 3

| Effect of HCII[1] on various component values in plasma of rats to which endotoxin is administered | | | | | |
|---|---|---|---|---|---|
| Component | Normal rat | Saline Group | Group H | Group A | Group H/A |
| Hyaluronic acid | 0.345[2] | 0.737 | 0.514 | 0.600 | 0.434 |
| TNFα | 0[3] | 383 | 169 | 240 | 150 |
| Keton body acid | 1.22 | 0.41 | 0.85 | 0.70 | 0.90 |
| Fibrinogen | 2.1[4] | 0.8 | 1.7 | 1.4 | 1.8 |
| GOT | 40[5] | 920 | 150 | 250 | 130 |

[1] Values at 18 hours after administration of LPS only with GOT, and at 3 hours with others.
[2] Absorbancy (wave length: 492 nm)
[3] Unit: pg/mℓ
[4] Unit: g/L
[5] Unit: Karmen unit

Experiment 3. Effect of HCII dose on its effect of inhibiting thrombus in organ

62.5, 125, 250 or 500 unit/kg-body-weight of HCII or 125, 250 or 500 unit/kg-body-weight of ATIII was solely administered to rats then LPS was administered to the rats with the method in Experiment 2 above, and the radioactivity per unit weight of liver was measured. The thrombus formulation inhibition ratios in respective administration groups was calculated in accordance with the equation below (Equation 2) and compared with each other.

$$[\text{Thrombus formation inhibition ratio}] = 100 - \{[\text{Radioactivity per unit weight in each administration group}] / [\text{Radioactivity per unit weight in control group}]\} \times 100 \qquad [\text{Equation 2}]$$

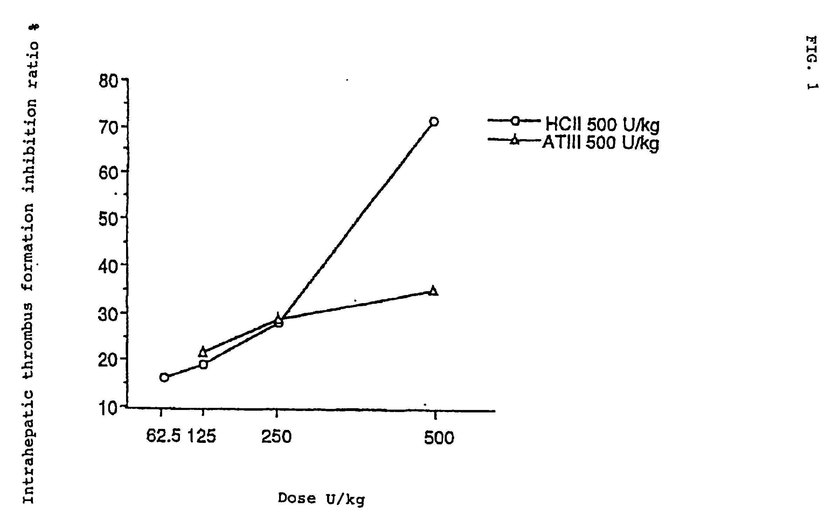

The results are shown in FIG. 1. The thrombus formation inhibition ratio in the liver increased slowly with the increase of the dose of administration both with HCII and ATIII. While the thrombus formation inhibition ratio stayed around 30% by administration of 500 unit/kg-body-weight of ATIII, the thrombus formation inhibition ratio reached about 70% by administration of 500 unit/kg-body-weight of HCII.

Experiment 4. Effect of improving organ vascular flow

(1) SD rats (male, 7-8 week of age) were separated into three groups (Group H, A and H/A) and HCII (500 unit/kg-body-weight), ATIII (500 unit/kg-body-weight) and mixed solution of HCII and ATIII (250 unit/kg-body-weight each) were intravenously administered respectively in Group H, A and H/A, then LPS (5 mg/kg-body-weight) was administered intravenously and the rats were served for following procedures.

(2) PE-5C catheters were inserted, one via the right femoral artery and another via the right common artery, to the left ventricle of these rats under anesthetic with 1% halothane, and both the catheters were passed through back hypodermis and indwelled in the posterior region of neck. The insertion of the catheter to the left ventricle was performed by monitoring the wave form with a pressure transducer. They were left for at least 3 hours, then the volume of organ vascular flow was measured.

(3) The blood pressure and cardiac rate of the treated rats above were measured via the catheter indwelled in the femoral artery using a pressure transducer. Then, 100, 000 of $^{51}$Cr-labeled microspheres (diameter: 15.5+-0.1 micron, specific activity: 1480 MBq/g) were injected to the left ventricle for 30 seconds. The microspheres used were those dissolved with 0.5 m$\ell$ physiologic saline containing 0.1% Tween 80 and treated with sonication for at least 30 minutes for prevention of aggregation. The collection of arterial blood for control blood was performed for 1 minute with the rate of 0.458 m$\ell$/minute from 5 seconds before injecting microspheres using a pump available from Harvard, and the radioactivity (a) was measured with a gamma-counter. At 15 minutes after injection of microspheres, the rats were sacrificed by the intravenous administration of a lethal volume of pentobarbital, and the brain, lungs, heart, spleen, liver, kidney, pancreas, stomach, small intestine, large intestine, back skin and left femoral region skin were extirpated and the weight (b) and the radioactivity (c) of respective organs were measured. The blood flows were calculated from the equation below (Equation 3) and compared with each other.

$$[\text{Blood flow each organ}] = [\text{Radioactivity per unit weight of each organ } (=c/b)] / [\text{Radioactivity per unit volume of control blood } (=a/0.458)] \qquad [\text{Equation 3}]$$

As a result, as shown in Table 4, the improvement of blood flow compared with Saline Group was recognized in all the organs in all the 3 administration groups, and the effects were more remarkable in the order of Group H/A > Group H > Group A.

Table 4

| Effect of HCII on fluctuation of organ vascular flow in rats to which endotoxin is administered | | | | | |
|---|---|---|---|---|---|
| Organ | Normal rat | Saline Group | Group H | Group A | Group H/A |
| Brain | 150[1] | 120 | 140 | 130 | 145 |
| Heart | 35 | 28 | 32 | 30 | 33 |
| Kidney | 20 | 10 | 15 | 13 | 17 |
| Liver | 3100 | 2200 | 2820 | 2512 | 2991 |
| Spleen | 80 | 42 | 68 | 60 | 71 |
| Pancreas | 142 | 75 | 101 | 88 | 112 |
| Lung | 200 | 132 | 172 | 160 | 180 |
| Stomach | 150 | 90 | 121 | 111 | 135 |
| Small intestine | 54 | 29 | 42 | 36 | 48 |
| Large intestine | 128 | 62 | 102 | 96 | 115 |
| Back skin | 820 | 500 | 680 | 613 | 708 |
| Left femoral region skin | 810 | 503 | 685 | 600 | 700 |

[1] Unit: $m\ell/g \cdot$ minute

Experiment 5. Effect of maintaining phagocytic activity of Kupffer cell

Kupffer cells locate in the liver sinusoid, form the sinusoid wall together with endothelial cells, and are characterized by a vigorous foreign body phagocytic activity. The phagocytic activity of Kupffer cells in the LPS administered rat prepared in accordance with a experimental procedure similar to Experiment 4 (1) above was observed by the following method to investigate the effect of administration of HCII.

India ink (80 mg/m$\ell$) 0.5 m$\ell$ was intravenously administered to a rat in respective drug administration groups 3 hours after administration of LPS. Blood was collected at 5 minutes and 20 minutes after the administration of India ink, and the degree of clearance of India ink was obtained from the difference in blood India ink concentration between the both. The blood India ink concentration was determined by measuring the absorbancy at 660 nm after hemolysis.

As a result, 96% of administered India ink was phagocytized in normal rats, but only 56% was phagocytized in the LPS administered rats in Saline Group. In contrast, 80, 72 and 85% were phagocytized respectively in Group H, A and H/A, revealing that the phagocytic activity of Kupffer cells are maintained by administration of respective drugs. This indicates, together with the function improving effect of sinusoid endothelial cells observed in Experiment 2, that HCII is effective for the function improvement of the liver sinusoid.

Experimental example 2. Effect of HCII administered to postoperative thrombosis model

Rabbits (Japanese white rabbit, male, 3-4 months of age) were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, A and H/A, then osteosynthesis in the thigh bone was performed. The rabbit was exposed to a constant pressure (300 hPa) for 40 hours, then the presence or absence of deep vein thrombus formation was judged by phlebography.

As a result, only 5 and 7 out of 20 rabbits were recognized to have thrombus formation in Group H and A, respectively, while 10 out of 20 rabbits were recognized to have thrombus formation in Saline Group, revealing that HCII and ATIII have a postoperative thrombus formation inhibition effect. In addition, only 3 out of 20 rabbits were recognized to have thrombus formation in Group H/A, indicating the effect of combined use of the both drugs.

Experimental example 3. Effect of HCII administered to thrombosis model associated with arteriopachynsis

Experiment 1. Antithrombotic effect

A pulse Doppler blood flowmeter was set to the femoral artery of Guinea pigs (Hartley strain, male, 8-10 weeks of age) under anesthetic with pentobarbital. These were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, Group A and Group H/A after the stability of vascular flow. After 15 minutes, rose bengal (10 mg/kg-body-weight) was administered intravenously and thrombus was formed by further radiating a green light at 540 nm in the blood vessel at the location of radiation. The thrombus formation was judged by the cessation of arterial vascular flow and the time required for the cessation of vascular flow was measured and compared to evaluate the antithrombotic effect of HCII.

As a result, the times required for the cessation of vascular flow in Group H, A and H/A were respectively 16, 10 and 20 minutes while the time was 7 minutes in Saline Group on the average. This revealed that HCII has an inhibitory effect also with the thrombus formation associated with blood vessel failure, that HCII has a greater inhibitory effect than ATIII and that the inhibitory effect further increases by the combined use with ATIII.

Experiment 2. Effect of inhibiting angiopachynsis

Thrombi were formed in Guinea pigs whom femoral artery a pulse Doppler blood flowmeter was set to with a similar method in Experiment 1 by rose bengal (10 mg/kg-body-weight) and the stimulus of a green light after stability of the vascular flow. The Guinea pigs were separated into three groups (Group H, Group A and Group H/A) and at 30 minutes after the formation of thrombi HCII (500 unit/kg-body-weight), ATIII (500 unit/kg-body-weight) and a mixed solution of HCII and ATIII (250 unit/kg-body-weight each) were intravenously administered respectively to Group H, A and H/A. From 15 minutes after the formation of thrombi, t-PA was continuously injected for 30 minutes (1 mg/kg-body-weight). After closing the aperture, the Guinea pigs were fed under usual conditions, and after 3 weeks, the thrombus formed part was extirpated and the areas of its lumen, intima and tunica media were measured, and the proportion of intimapachynsis was calculated from these results and compared to evaluate the effect of HCII to inhibit angiopachynsis.

As a result, the average intimapachynses in Group H, A and H/A were respectively 1.38, 1.89 and 1.06 ($\times 10^{-2}$mm$^{-2}$) while the average intimapachynsis in Saline Group was 2.36 ($\times 10^{-2}$mm$^{-2}$), indicating that HCII has also the effect of inhibiting angiopachynsis. The angiopachynsis inhibition effect was also recognized with ATIII, but HCII was more effective. The combined use of HCII and ATIII further increased the inhibitory effect. A similar experiment performed by administering urokinase or SOU-PA ($10^5$ unit /kg and $10^6$ IU/kg, respectively) as plasminogen activator resulted in results similar to those above in either case.

From the results above, it was indicated that the administration of HCII is effective for such diseases as acute occlusion and restenosis after PTCA or PTCR. Reference example 2. Fluctuation of blood HCII in incomplete peripheral circulation model

SD rats (male, 7-8 weeks of age) were anesthetized with Nembutal, then fixed with face-up position and their right femoral regions were exposed. After blocking vascular flow with forceps, 1 or 2 mg/leg of rauric acid was injected to a site more peripheral than femoral artery. The ischemic interval at the injection of rauric acid was 1-2 minutes. A physiologic saline was administered instead of lauric acid in a control. After covering the injection hole with an instantaneous adhesive agent, the skin aperture was closed. Specimen plasma was collected by the citric acid blood collection from cervical vein before, 3 hours, and 1, 3 and 7 days after administration.

After dispensing 50 $\mu\ell$ of TRIS hydrochloride buffer containing dermatan sulfate to a microplate, 3.5 $\mu\ell$ of specimen plasma or pooled plasma was added. 100 $\mu\ell$ of human thrombin solution was further added and the mixture was agitated, allowed to stand for 5 minutes at the room temperature, then 100 $\mu\ell$ of synthetic substrate (S-2238) was added. The absorbancy after a constant time was measured, and the concentration of HCII in the specimen plasma was calculated as percentage to the concentration of HCII in the pooled plasma.

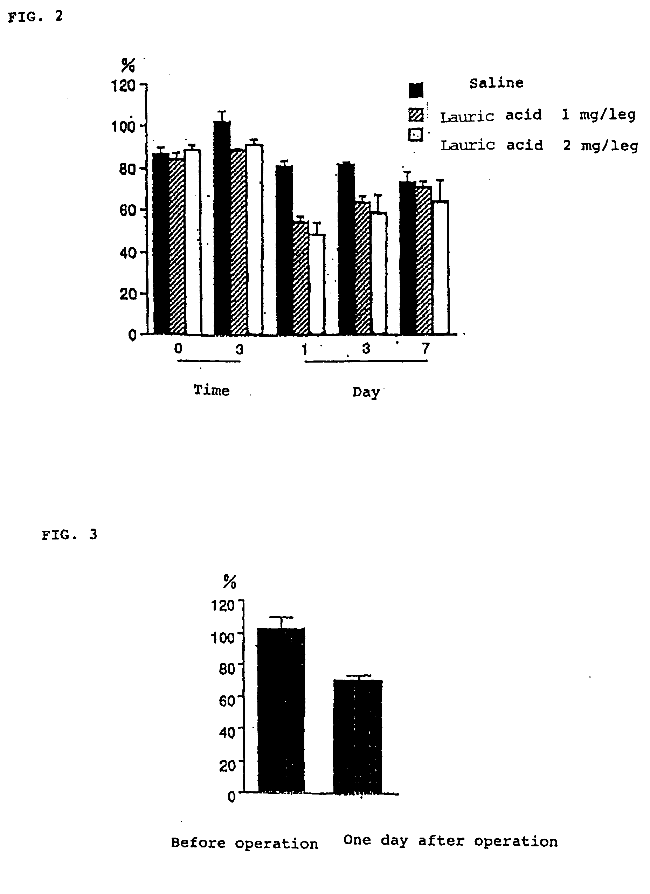

As a result, a significant reduction of the blood HCII level was recognized with the administration of lauric acid (FIG. 2). The effect was most prominent at 1 day after the administration and it continued till at least 3 days after the administration. Experimental example 4. Effect of HCII administered to incomplete microcirculation model

Following the method in Reference example 2, the rats to which lauric acid was administered were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, A and H/A once daily for 7 days from the day of lauric acid administration. The lesions of legs at 7 days after the administration of lauric acid were observed with naked eyes. The degree of lesion was separated into five steps from normal 0 to the most severe 4 to evaluate the lesion inhibitory effect of HCII.

As a result, the average lesion scores in Group H, A and H/A were respectively 1.2, 1.6 and 0.8 while the average lesion score in Saline Group was 3.0, indicating that HCII and ATIII have an effect of inhibiting the lesion. Particularly, the combined use of both the drugs was effective. It was suggested from this fact that HCII is effective for Buerger's disease, angiitis such as chronic arterial obstruction, incomplete microcirculation concerned by thrombus or blood vessel constriction, the disease associated with other blood vessel failures and the disease associated with inflammation.

It was revealed in the course of performing the experiment in Reference example 2 that the reduction of blood HCII is observed at ischemia [described in detail in (1) in Reference example 3 below].

Reference example 3. Fluctuation of blood HCII in ischemic disease model

(1) Femoral arterial ischemia delegation model

This model is of the control group for the lauric acid administered rat of incomplete peripheral circulation model described in Reference example 2, to which 0.1 m$\ell$/extremity of physiologic saline is administered instead of lauric acid. The blocking vascular flow with forceps at the time of injecting physiologic saline made an ischemic state for 1-2 minutes. It was recognized that the blood HCII level after the ischemia was lower than before (Saline Group in FIG. 2).

(2) Kidney blood vessel delegation model

SD rats (male, 7-8 weeks of age) were anesthetized with Nembutal, then their abdominal cavity was opened, the left kidney aorta and large vein and the part of large vein near the left kidney were removed from the peripheral tissue and the branch blood vessel was delegated. The left kidney vein was clipped with forceps or vascular clip to block the vascular flow and the rats were left for 10-30 minutes. Specimen plasma was collected by the citric acid blood collection from cervical vein before and one day after the operation and the blood HCII level was measured (FIG. 3; calculated as percentage of the HCII level before the operation). As a result, the blood HCII level was observed to decrease after the operation i.e. after the ischemia.

Experimental example 5. Effect of HCII administered to ischemic disease model

Next, the effect of administration of HCII against an ischemic disease was investigated. SD rats (male, 9-10 weeks of age) were anesthetized by administering 350 mg/kg-body-weight of chloral hydrate into the abdominal cavity, then catheters were inserted to the cervical vein. To reduce the average arterial blood pressure to 50 mmHg, 5 m$\ell$ of blood was collected through the catheter in the cervical vein. The rats were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, A and H/A. The prepared cervical vein was disposed, and was clipped with forceps as soon as the blood was collected. The forceps were removed after 30 minutes, and the collected blood was injected again (reperfusion). At the time of the reperfusion, respective agents were additionally administered to the rats in the corresponding groups with a way similar to that before starting ischemia. A fibrin sealant [Tissel (TM)] was applied to the injury. The animals were in an anesthetic state for 24 hours in total (23.5 hours after the reperfusion). Respective groups were divided into two and were served for Experiment 1 and 2 below.

Experiment 1. Evaluation of brain edema and brain blood flow

The brain blood flow of the treated rats above which were kept at a constant temperature for 24 hours was measured in total using a Doppler blood flowmeter, then the rats were euthanized with ether, and the brain was extirpated. The evaluation of brain edema was performed in accordance with the method published for other species (Refer to Oh & Betz, Stroke 22: 915-921, 1991).

Water content, which is a parameter to evaluate the brain edema induced by ischemia/reperfusion, was determined as follows.

The wet weights of the both hemispheriums were measured, and after drying at 200°C for 17 hours, the weights (dry weight) were measured again. The water content was obtained by substituting the respective measurements for the equation below (Equation 4).

$$\text{Water content (\%)} = \frac{\text{(wet weight) - (dry weight)}}{\text{(wet weight)}} \times 100 \qquad \text{[Equation 4]}$$

As a result, the water content of the brain in the respective drug administration groups indicated lower values than the physiological saline group (Table 5). An improvement of brain blood flow was also recognized in the respective drug administration groups.

Table 5

| Effect of HCII on cerebral failure in ischemia induction rats | | | | | |
|---|---|---|---|---|---|
| | Parameter | Saline Group | Group H | Group A | Group H/A |
| Brain water content | 83.6 | 85.0 | 84.1 | 84.3 | 83.9 |
| Neurogic absence score | 5 | 20 | 12 | 14 | 8 |
| Brain blood flow (ml/minute /100 g) | 30 | 10 | 17 | 16 | 20 |

Experiment 2. Evaluation of neurologic absence

Respective symptoms were made into scores with the state of consciousness, ambulation, muscle tone and doing behavior after operation (i.e. after recovery of consciousness), 1st, 2nd and 3rd day, and the neurologic absence was evaluated based on the cumulative scores for the three days (the higher the cumulative scores, the larger the neurologic absence). As a result, the neurologic absence scores in Group H, Group A and Group H/A were respectively 12, 14 and 8 while that in Saline Group was 20 (Table 5).

It was revealed from the results in Experiment 1 and 2 above that both the drugs of HCII and ATIII are effective for ischemic diseases, particularly ischemic brain disorder.

Experimental example 6. Effect of HCII administered to brain disorder model

Experiment 1. Effect of administration of HCII in lung disorder model of rats administered LPS

(1) LPS 2 mg/kg-body-weight was administered intravenously to SD rats (male, 7-8 weeks of age) and after 24 hours, $^{125}$I-human serum albumin was intravenously administered. The rats were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, Group A and Group H/A, then LPS was administered again. All the blood was drawn after 3 hours and the lungs were extirpated, and the wet weight and radioactivity were measured. With all the groups, the lungs of some of the rats were fixed with formalin and observed with a light microscope.

As a result, the average lung weights in Group H, A and H/A were respectively 1.38, 1.44 and 1.32 g while the average lung weight in Saline Group was 1.53. The values in former three groups were closer to 1.22 g of a normal rat than that in the latter. The lung radioactivity in Group H, A and H/A were respectively 2806, 3312 and 2010 cpm/g while the lung radioactivity in Saline Group was 7630 cpm/g. These results suggest that the formation of edema and the accentuation of membrane permeability are inhibited by the administration of HCII or ATIII.

The histological investigation of the lungs with a light microscope resulted in the recognition of obvious increase of the invasion of inflammatory cells (blood neutrophil) to the lungs in Saline Group. On the other hand, the decrease of the invasion of inflammatory cells was recognized in respective drug administration groups.

(2) Rats were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were continuously injected for 48 hours respectively to Group H, A and H/A together with LPS. The arterial blood was collected and gas analysis was performed.

As a result, the arterial blood oxygen partial pressures in Group H, A and H/A were respectively 74, 70 and 78 mmHg while that in Saline Group was 60 mmHg on the average. It was suggested that the improvement of lung disorder was observed in the respective drug administration groups since the higher oxygen partial pressure indicates more oxygen supply to hemoglobin.

From the results above, it was shown that HCII has a lung disorder improving effect.

Experiment 2. Effect of HCII administered to goat immature new-born baby oxygen disposer model

The immature new-born babies extracted from pregnant goats with Caesarean operation were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, A and H/A, then they were disposed to oxygen of a high concentration with an artificial ventilation, and the alveolar-arterial oxygen difference (AaDO$_2$) was measured by a gas analysis after 4 hours. Then the lungs were extirpated, fixed in the usual way, then they were investigated histologically.

As a result, the AaDO$_2$ in Group H, A and H/A were respectively 290, 340 and 265 mmHg while the AaDO$_2$ in Saline Group was 578 mmHg on the average, showing an decrease of partial pressure difference. The histological investigation resulted in observance of hyaline membranes in the lungs in respective groups, but the symptoms in the respective drug administration groups were all improved than those in Saline Group. Thus the improvement of histological findings by administration of HCII or ATIII was recognized.

Experimental example 7. Effect of HCII administered to nephrotic syndrome model

To SD rats (male, 7-8 weeks of age) 20 mg/kg-body-weight of puromycin was administered once daily for 4 days. The rats were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, A and H/A once daily from the day of the last administration of puromycin to 9 days after the day of the last administration. The ascites fluid volume and urinary protein on the 9th day after the day of the last administration were measured and evaluated with 6 steps of 0 to 5-.

As a result, the urinary protein levels in Group H, A and H/A were respectively 1+ to 3+, 2+ to 3+ and 1+ to 2+, while the urinary protein level in Saline Group was 4+ to 5+. The ascites fluid volume in the respective drug administration groups showed lower levels than those in Saline Group, indicating that the administration of HCII is effective for nephrotic syndrome. Experimental example 8. Effect of HCII administered to glomerular nephritis model

Experiment 1. Effect of administration of HCII in rats administered anti-thymic cell serum

Rat thymic glands were administered to rabbits, and anti-thymic cell serum was prepared in a usual way. To SD rats (male, 7-8 weeks of age) 10 m$\ell$/kg-body-weight of the anti-thymic cell serum was administered intravenously together with normal rabbit serum. The rats were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, A and H/A once daily from the day of administration of the anti-thymic cell serum to 6 days after the administration. The proportions of the extracellular matrix (ECM) to the total area of glomeruli were calculated as matrix scores and compared to each other.

As a result, the average matrix scores in Group H, A and H/A were 24, 35 and 17% while the average matrix score in Saline Group was 52%, showing that HCII has a inhibitory effect of glomerular nephritis.

Experiment 2. Effect of HCII administered to Masugi nephritis rabbit

The renal cortex of a rabbit (Japanese white rabbit, male, 3-4 months of age) was extirpated, homogenized and intraperitoneal sensitization of 10 m$\ell$ of the obtained homogenate (20% suspension) as rabbit renal antigen was performed in a domestic duck. Fifteen times of immunization in total was performed every 7 days and anti-rabbit renal antiserum was obtained. To rabbits (Japanese white domestic rabbit, male, 3-4 months of age) 2 m$\ell$/kg-body-weight of the domestic duck antiserum was intravenously administered to produce Masugi nephritis. The rabbits with 300 mg/d$\ell$ of urinary protein (not more than 150 mg/d$\ell$ with a normal rabbit) at 5 days after the administration of the antiserum were selected as nephritis crisis animals, which were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, A and H/A once daily for 11 days from that day. Whether the rabbits were alive or dead at 16 days after the administration of the antiserum was observed while urinary protein and blood urea nitrogen (BUN) were measured.

Table 6 shows the results. The numbers of surviving were 11, 10 and 12 rabbits respectively in Group H, A and H/A while only 5 out of 16 rabbits were alive in Saline Group, indicating the improvement by the administration of both the drugs of HCII and ATIII.

The urinary protein and BUN in the respective drug administration groups showed lower values than those in Saline Group, indicating that both the drugs are effective for Masugi nephritis.

Table 6

| Effect of HCII administered to Masugi nephritis rabbit | | | | |
|---|---|---|---|---|
| Parameter | Saline Group | Group H | Group A | Group H/A |
| Survival rate | 5/16 | 11/16 | 10/16 | 12/16 |
| Urinary protein (mg/dl) | 4450±350 | 2003±381 | 2198±280 | 1850±360 |
| BUN(mg/dl) | 91±26 | 56±16 | 62±15 | 47±11 |

Experimental example 9. Effect of HCII administered to pancreatitis model

Using a taurocholic acid administration rat which is a pancreatitis model, the effect of HCII against pancreatitis was investigated. SD rats (male, 7-8 weeks of age) were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, A and H/A. To the rats 5% of taurocholic acid mixed solution was injected into the pancreatic duct in a retrograde manner, and the variations of ascites fluid volume, pancreatic tissue lesion (necrosis and bleeding were respectively made into scores in 5 steps making normal to be 0 and the strongest lesion to be 4), blood TAT and D-dimer at 10 hours after the injection were measured. TAT and D-dimer were measured using commercially available kits. The survival rate after 3 days was also observed.

As a result, the numbers of surviving were 9, 8 and 12 rabbits respectively in Group H, A and H/A while only 3 out of 20 rabbits were alive in Saline Group, indicating the survival rate improving effect in the respective drug administration groups.

The averages of the ascites fluid volume in Group H, A and H/A were respectively 5.0, 6.8 and 4.0 m$\ell$, showing the decrease of pooling in comparison with 11.5 m$\ell$ in Saline Group. The average pancreatic tissue necrosis scores in Saline Group, Group H, A and H/A were respectively 2.7, 1.5, 2.0 and 1.0, the average bleeding scores in the same groups were respectively 1.9, 0.7, 1.1 and 0.4, indicating that both the drugs of HCII and ATIII have the pancreatic tissue lesion improving effect. With TAT and D-dimer, improvements were further recognized in the respective drug administration groups: the averages of TAT in Saline Group, Group H, A and H/A were respectively 8.0, 3.9, 5.1 and 2.8 ng/m$\ell$ and the averages of D-dimer in the same groups were respectively 32.0, 15.8, 23.2 and 10.7 ng/m$\ell$.

From the results above, it was revealed that HCII has disease condition improving effects against pancreatitis and pancreatic disorder. It was also suggested that HCII may be effective for preventing disseminated intravascular coagulation. Experimental example 10. Effect of HCII administered to inflammation model associated with formation of edema

Experiment 1. Effect of administration of HCII in carrageenin administration rats

The edema formation was induced by subcutaneously administering 50 $\mu\ell$/rat of 1% carrageenin to the hind leg of SD rats (male, 7-8 weeks of age). The rats were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were administered from the tail vein respectively to Group H, A and H/A, and the volume of edema was measured every 1 hour. The volumes of edema at respective times were compared as the percentages of the volume just after administration of carrageenin.

As a result, the volumes of edema at 3 hours after the administration of carrageenin in Group H, A and H/A stayed at respectively 125, 135 and 110% while that in Saline Group increased to 170%. Thus it was confirmed that both the drugs of HCII and ATIII have remarkable anti-inflammation effects.

Experiment 2. Effect of HCII administered to articular rheumatism model

Freund's complete adjuvant (FCA; 6.0 mg/m$\ell$ liquid paraffin for injection) was injected, 0.1 m$\ell$ to each SD rats (female, 7-8 weeks of age) in the right hind leg subcutaneously. The rats were separated into three groups (Group H, Group A and Group H/A) and HCII (500 unit/kg-body-weight), ATIII (500 unit/kg-body-weight) and a mixed solution of HCII and ATIII (250 unit/kg-body-weight each) were intravenously administered respectively to Group H, A and H/A once daily for 18 days from that day. The volume of the right and left hind legs were measured every second day until 18 days from the day of administering FCA. The edema (primary inflammation) in the adjuvant treated leg (right hind leg) in both Saline Group and the respective drug administration groups linearly enlarged until 6 days after the treatment, then the enlargement stopped. The edema (secondary inflammation) in the non-treatment leg (left hind leg) appeared from 12 days after the treatment and linearly enlarged thereafter. The edema rates (calculated as percentage of the volume of respective legs in Saline Group) of treated leg after 12 days and those of non-treatment leg after 18 days are shown in Table 7. The edema was decreased both with treated leg and non-treatment leg in respective drug administration groups. From the results, it was suggested that both the drugs of HCII and ATIII may be useful for articular rheumatism.

Table 7

| Effect of HCII administered to adjuvant articular rheumatism rat | | |
|---|---|---|
| Administration group | Edema rate(%) | |
| | Treated leg | Non-treatment leg |
| Saline Group | 100 | 100 |
| Group H | 80 | 82 |
| Group A | 84 | 85 |
| Group H/A | 77 | 82 |

The medicinal composition having HCII as active ingredient and the medicinal composition above prepared by compounding HCII and AT III of the present invention are extremely effective for prevention and therapy of sepsis, thrombosis, incomplete microcirculation, ischemic disease, organ derangement in liver, lungs, kidney, brain, etc. and various inflammations.

**Claims**

1. A pharmaceutical composition comprising heparin cofactor II and optionally a pharmaceutically acceptable carrier.

2. The pharmaceutical composition of claim 1 further comprising antithrombin III.

3. Use of heparin cofactor II for the preparation of a pharmaceutical composition for the prevention or treatment of sepsis.

4. Use of heparin cofactor II for the preparation of a pharmaceutical composition for the prevention or treatment of a disease associated with abnormal vascular flow.

5. The use of claim 4, wherein said disease is thrombosis.

6. The use of claim 4, wherein said disease is incomplete microcirculation.

7. The use of claim 4, wherein said disease is an ischemic disease.

8. Use of heparin cofactor II for the preparation of a pharmaceutical composition for the prevention or treatment of derangement of organs selected from the group consisting of liver, lungs, kidney and brain.

9. Use of heparin cofactor II for the preparation of a pharmaceutical composition for the prevention or treatment of an inflammation.

10. The use of claim 9, wherein said inflammation is selected from the group consisting of glomerular nephritis, pancreatitis, rheumatism and an inflammation associated with edemaplasia.

11. The use of any one of claims 3 to 10, wherein said composition is for administration in an amount of 1-10000 unit/kg-body-weight per day.

12. The use of any one of claims 3 to 11, wherein said composition is in the form for a route of administration selected from the group consisting of intravenous administration, intraarterial administration, and inhalation.

**13.** The use of any one of claims 3 to 12, wherein the pharmaceutical composition further comprises antithrombin III.

FIG. 1

FIG. 2

FIG. 3